# EUROPÄISCHE PATENTANMELDUNG

(11) **EP 1 543 878 A2**
(43) Veröffentlichungstag der Anmeldung: **22.06.2005**
(21) Anmeldenummer: 04029502.4
(22) Anmeldetag: 14.12.2004
(51) Int. Cl.: B01L 3/00, G01N 33/48

(54) **Testelement zur thermostatisierten Untersuchung von Probenmaterial**

(30) Priorität: 16.12.2003 DE 10359160
(71) Anmelder: F. Hoffmann-La Roche AG, 4070 Basel (CH); Roche Diagniostics GMBH, 68305 Mannheim (DE)
(72) Erfinder: Ocvirk, Gregor, Dr., 68239 Mannheim (DE); Kalveram, Stefan, Dr., 68519 Viernheim (DE); Roesicke, Bernd, 68305 Mannheim (DE)
(74) Vertreter: Pfiz, Thomas, Dr.

(57) **Zusammenfassung**

Die Erfindung betrifft Testelement zur Untersuchung von Probenmaterial wie Blut oder Urin mit einem Testträger (12), welcher einen mit dem Probenmaterial beaufschlagbaren Untersuchungsbereich (18) aufweist, und einem mit dem Untersuchungsbereich (18) in Wärmeleitkontakt stehenden Heizelement (22). Erfindungsgemäß wird vorgeschlagen, dass das in den Testträger (12) integrierte Heizelement durch einen unter Stromdurchfluss eigenerwärmten, auf eine vorgegebene Zieltemperatur selbstregelnden Thermistor (22) gebildet ist.

## Beschreibung

Die Erfindung betrifft ein Testelement zur Untersuchung von Probenmaterial wie Blut oder Urin mit einem Testträger, welcher einen mit dem Probenmaterial beaufschlagbaren Untersuchungsbereich aufweist, und mit einem Heizelement, das mit dem Untersuchungsbereich in Wärmeleitkontakt steht.

Die Verwendung von disposiblen Teststreifen in tragbaren Handgeräten und von Einwegkartuschen in stationären Messgeräten ermöglicht die zeitgerechte und kostengünstige Bestimmung von analytischen Parametern wie Blutgasen und -elektrolyten, unterschiedlichen Metaboliten wie Glucose in Vollblut, Serum, Gewebeflüssigkeit oder Urin und auch die Bestimmung von Blutgerinnungswerten für die Einstellung von Gerinnungshemmern. In der Mehrzahl der genannten Testelemente erfolgt zumindest eine spezifische Umsetzung eines Reagens mit dem interessierenden Analyten in einem begrenzten Untersuchungsbereich. Dabei ist die Reaktionsgeschwindigkeit und somit auch das Messergebnis nach einer bestimmten Reaktionszeit von der Temperatur am Messfeld abhängig. Eine hohe Reproduzierbarkeit und Genauigkeit der Analyseresultate ist jedoch eine wesentliche Voraussetzung für die Ableitung therapeutischer Maßnahmen.

In diesem Zusammenhang ist es bekannt, den Testträger durch einen geräteseitigen Heizer zu temperieren, so dass vor der Messung die gewünschte Temperatur eingestellt werden kann. Hierfür muss jedoch eine ausreichend lange Heizperiode der eigentlichen Messung vorgeschaltet werden, wodurch sich die Messzeit und der Energiebedarf erhöhen. Auch aufgrund der erforderlichen Positioniergenauigkeit ist es schwierig, kleine Testfelder lokal zu erwärmen.

Auf dem eher entfernten Gebiet der PCR(polymerase chain reaction)-Technik wurde in der US 6,312,886 die direkte Heizung von Reaktionsgefäßen vorgeschlagen, um verschiedene Temperaturen anzusteuern. Dort sind in die Probencontainer integrierte elektrisch leitende Polymerschichten beschrieben, die unter Stromdurchfluss Wärme abgeben. Die Stromversorgung erfolgt über externe Steuereinrichtungen, die den Geräteaufwand beträchtlich erhöhen.

Ausgehend hiervon liegt der Erfindung die Aufgabe zugrunde, die im Stand der Technik aufgetretenen Nachteile zu vermeiden und als Verbrauchsmaterial vorgesehene Testelemente der eingangs angegebenen Art dahingehend zu verbessern, dass bei kostengünstiger Fertigungsmöglichkeit mit geringem apparativem Aufwand reproduzierbare Messbedingungen sichergestellt sind.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen Temperaturregelkreis mit inhärenter Führungsgröße in den Testträger zu integrieren. Dementsprechend wird erfindungsgemäß vorgeschlagen, dass das in den Testträger integrierte Heizelement durch einen unter Stromdurchfluss eigenerwärmten, auf eine vorgegebene Zieltemperatur selbstregelnden Thermistor gebildet ist. Der Thermistor erhitzt sich weitgehend unabhängig von der Umgebungstemperatur auf eine Ziel- bzw. Gleichgewichtstemperatur, bei der die zugeführte elektrische Leistung gleich der abgegebenen Wärmeleistung ist. Sinkt die Temperatur, nimmt der Thermistor wegen seines verringerten Widerstandes mehr elektrische Leistung auf und die Temperatur steigt wieder an. Umgekehrt nimmt bei Übertemperatur der Widerstand sprungartig zu, so dass sich die Stromstärke entsprechend verringert. Damit ist es möglich, eine selbstregulierende Heizung direkt auf dem Testelement zu realisieren, ohne dass Temperaturfühler und geräteseitige Heiz- und Regeleinrichtungen erforderlich wären. Somit verringern sich auch die Gerätebaugrößen und -kosten. Durch die gemeinsame Integration von Heiz- und Testfeld in das Testelement wird eine gezielte Temperierung mit geringem Energiebedarf möglich, wobei externe Wärmeübergangswiderstände entfallen. Dadurch ist auch eine hohe Konstanz der Testtemperatur erreichbar.

Vorteilhafterweise besitzt der Thermistor als Kaltleiter bei zunehmender Temperatur im Bereich der Zieltemperatur einen sprunghaften, nichtlinearen Widerstandsanstieg, so dass eine exakte Temperaturbegrenzung möglich ist.

Um den Wärmeaustausch mit dem Untersuchungsfeld zu optimieren, ist es von Vorteil, wenn der Thermistor als Heizfeld, vorzugsweise als Dünnschichtheizfeld ausgebildet ist. Dies lässt sich dadurch realisieren, dass der Thermistor vorzugsweise durch ein Beschichtungs- oder Druckverfahren als Flächengebilde in den Testträger integriert ist.

Eine vorteilhafte Ausführung sieht vor, dass der Thermistor aus einem Kompositwerkstoff bestehend aus einem Bindemittel und darin eingebrachten elektrisch leitfähigen Bestandteilen gebildet ist. Für die gewünschte Eigenregulierung ist es von Vorteil, wenn der Kompositwerkstoff bei der Zieltemperatur einen die elektrische Leitfähigkeit beeinflussenden Phasenübergang durchläuft.

Gemäß einer weiteren vorteilhaften Ausgestaltung besteht das Bindemittel aus Monomeren oder Polymeren, während die leitfähigen Bestandteile vorteilhafterweise durch Rußpartikel, Kohlenstofffasern, Metallfäden oder leitfähige Polymerteilchen gebildet sind.

In bevorzugter Verwendung ist der Testträger als Einwegteil für Einmaluntersuchungen vorgesehen ist.

Für eine vereinfachte Herstellung und Anwendung ist es von Vorteil, wenn der Testträger durch ein flächiges Substrat, insbesondere einen vorzugsweise als Folienverbundteil ausgebildeten Teststreifen gebildet ist.

Um die erzeugte Wärme gezielt zu nutzen, ist es günstig, wenn der Untersuchungsbereich zumindest teilweise durch den Thermistor begrenzt oder mit diesem über eine Zwischenfolie thermisch leitend verbunden ist. Es versteht sich, dass hierbei der Untersuchungsbereich und der Thermistor eine ausreichende Wärmeleitfähigkeit aufweisen sollten.

Vorteilhafterweise ist der Untersuchungsbereich durch ein mit Trockenchemikalien beschichtetes, auf einen Analyten in dem aufgebrachten flüssigen Probenmaterial ansprechendes Reaktionsfeld gebildet. Zur Vorerwärmung des Probenmaterials ist es von Vorteil, wenn sich der Thermistor über den Untersuchungsbereich hinaus auf einen Probenzuführkanal des Testträgers erstreckt.

Für die Testauswertung ist es vorteilhaft, wenn der Thermistor zugleich einen Temperaturfühler zur Bestimmung der Untersuchungstemperatur über eine Widerstandsmessung bildet.

Die Energieversorgung kann dadurch erfolgen, dass an dem Testträger mit dem Thermistor verbundene, vorzugsweise durch Leiterbahnen gebildete Anschlüsse für eine geräteseitige Spannungsquelle angeordnet sind.

Für Biotests ist es vorteilhaft, wenn die Zieltemperatur im Bereich zwischen 25 bis 50°C, vorzugsweise 30 bis 40°C liegt, mit einer Abweichung vom Sollwert von weniger als 1°C.

Weiterhin ist es für die Reduzierung der Testdauer und des Energiebedarfs von Vorteil, wenn die gesamte Heizzeit weniger als 5 Minuten beträgt, und wenn der Thermistor nur ein Teilvolumen des Probenmaterials, welches sich in Kontakt mit dem Untersuchungsbereich und gegebenenfalls in einem einströmseitig angrenzenden Abschnitt des Probenzuführkanals befindet, direkt erwärmt.

Gegenstand der Erfindung ist auch ein Messgerät, insbesondere portables Blutzucker- oder Blutgerinnungsmessgerät zur Verarbeitung von selbsttemperierenden Testelementen.

Im Folgenden wird die Erfindung anhand eines in der Zeichnung in schematischer Weise dargestellten Ausführungsbeispiels näher erläutert. Es zeigen
- Fig. 1: ein Blockschaltbild eines portablen Blutzuckermessgeräts mit einem einschiebbaren Testelement;
- Fig. 2 und 3: eine perspektivische Zusammenbau- und Explosionsdarstellung des Testelements; und
- Fig. 4: das Testelement im Querschnitt.

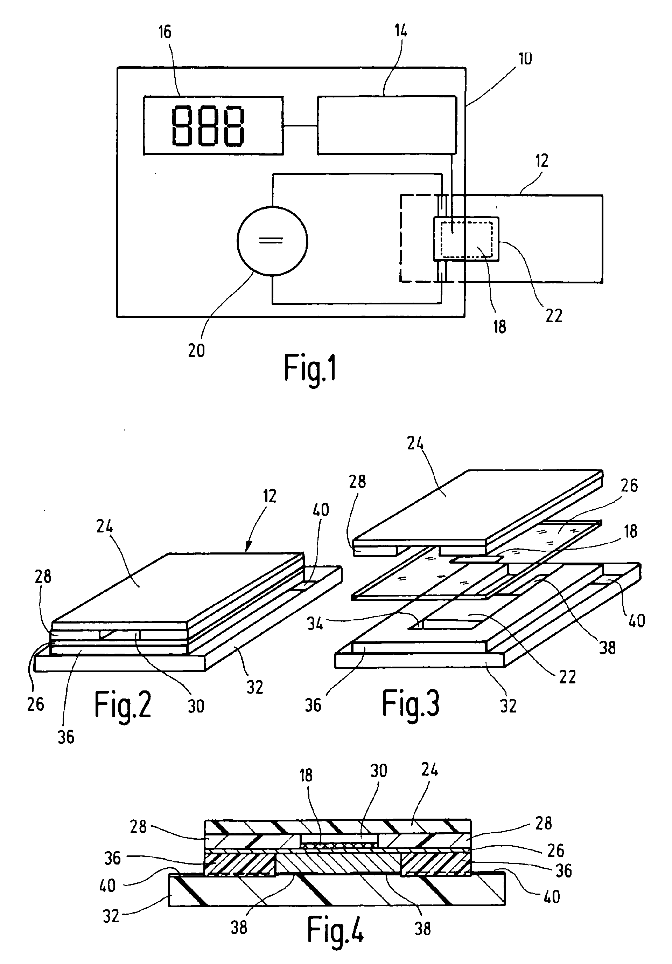

Das in Fig. 1 dargestellte portable Blutzuckermessgerät 10 ermöglicht die Verarbeitung eines disposiblen streifenförmigen Testelements 12 mittels einer beispielsweise photometrisch oder elektrochemisch arbeitenden Messund Auswerteeinheit 14 sowie die Anzeige des Ergebnisses auf einer Anzeigeeinheit 16. Das Testelement 12 weist ein mit Blutflüssigkeit beaufschlagbares Untersuchungsfeld 18 auf, das mit einem aus der geräteseitigen Gleichspannungsquelle 20 gespeisten Thermistor 22 als PTC-Heizelement auf eine vorgegebene Zieltemperatur selbstregelnd temperierbar ist.

Wie am besten aus Fig. 2 und 3 ersichtlich, besteht das für Einmaluntersuchungen bestimmte Testelement 12 als Testträger-Verbundteil aus mehreren Folienlagen. Zwischen einer Deckfolie 24 und einer Zwischenfolie 26 ist über einen längsgeteilten Abstandshalter 28 ein kapillarer Probenzuführkanal 30 freigehalten, der zu dem Untersuchungsfeld 18 auf der Zwischenfolie 26 führt.

Unterhalb der Zwischenfolie 26 ist gegenüber einer beidseitig überstehenden Bodenfolie 32 eine Heizkammer 34 durch einen ausgeschnittenen Abstandshalter 36 begrenzt. In der Heizkammer 34 ist der Thermistor 22 als Flächengebilde so integriert, dass über die gut wärmeleitende Zwischenfolie 26 eine flächige Wärmeleitverbindung zu dem Untersuchungsfeld 18 besteht. Dabei erstreckt sich der Thermistor 22 im wesentlichen über das Untersuchungsfeld 18 und noch darüber hinaus in den Bereich des Probenzuführkanals 30, um eine Vorerwärmung des einströmenden Probenmaterials zu ermöglichen. Der Einlass und Auslass des Probenzuführkanals 30 werden allerdings nicht direkt beheizt, um eine Zunahme der Verdunstungsrate des Probenmaterials zu vermeiden. Durch die Erwärmung nur eines Teilvolumens der Probe kann die Heizzeit auf wenige Minuten reduziert werden. Zur elektrischen Kontaktierung des Thermistors 22 sind auf die Bodenfolie 32 beispielsweise durch Siebdruck Leiterbahnen 38 aufgebracht, welche in seitlich freiliegenden Anschlussfahnen 40 enden.

Das Untersuchungsfeld 18 ist durch eine mit Trockenchemikalien versehene Reaktionsschicht gebildet, die auf einen Analyten (Glucose) in der Blutflüssigkeit unter Farbveränderung anspricht. Dies lässt sich durch die transparente Deckfolie 24 hindurch mittels der Messeinheit 14 photometrisch erfassen.

Der Thermistor 22 besteht als Kompositwerkstoff aus einem leitfähigen Gemisch beispielsweise aus einem Monomer wie Zimtsäuremethylester, 1,6 Hexandiol oder Nicontinsäuremethylester einerseits und leitfähigen Partikeln wie Rußteilchen von beispielsweise 300 nm Teilchengröße und 10 m²/g Oberfläche andererseits. Der Füllstoffanteil beträgt vorzugsweise weniger als 60%, so dass ein Verhältnis von Kalt- zu Heißwiderstand größer 1:100 dargestellt werden kann, das Temperaturverhalten nur eine geringe Hysterese zeigt und der Kompositwerkstoff vor Auftrag ausreichend dispergiert werden kann.

Neben dem Anteil des Füllstoffes ist es wesentlich, dass die Füllstoffpartikel nach Erhitzen und Überschreitung eines Phasenübergangspunktes hinreichend voneinander getrennt im Kompositwerkstoff vorliegen, um einen ausreichend hohen Heißwiderstand zu gewährleisten. Daher ist die spezifische Oberfläche vorzugsweise geringer als 10 m²/g zu wählen. Um eine schnelle Erwärmung des Untersuchungsfeldes 18 auf die gewünschte Temperatur zu erreichen, ist es vorteilhaft, den Kaltwiderstand des Thermistors bei gegebener Nennspannung gering zu halten. Bei gegebenem Anteil an elektrisch leitfähigen Partikeln kann der Kaltwiderstand durch sorgfältige Dispersion des Kompositwerkstoffes bei über dem Schmelzpunkt liegenden Temperaturen und durch Einbringen von Ultraschall vor Auftrag deutlich verringert werden. Zusätzlich kann der Kaltwiderstand des wie oben beschrieben dargestellten Heizfeldes durch eine ausreichend lange Abkühlzeit und durch Umkristallisation vor Auftrag des geschmolzenen leitfähigen Gemisches gering gehalten werden.

Das Heizfeld kann durch Auftragen einer dünnen Schicht des erhitzten leitfähigen Gemisches auf den mit Leiterbahnen 38 versehenen Bereich der Heizkammer 34 hergestellt werden, so dass bei Abkühlung eine gleichmäßige Kristallisation eintritt und ein reproduzierbarer elektrischer Kontakt zwischen den Leiterbahnen und dem leitfähigen Gemisch gewährleistet ist. Hierfür sind speziell Graphitleiterbahnen und im allgemeinen alle Leiterbahnen einsetzbar, die mit hoch reproduzierbaren Widerstandswerten herstellbar sind und durch Auftrag des Kompositwerkstoffes reproduzierbar kontaktiert werden können.

Der so gebildete Thermistor 22 zeigt als Kaltleiter im Bereich einer Ziel- bzw. Umschalttemperatur einen sprunghaften, nichtlinearen Widerstandsanstieg.

Aufgrund der für analytische Bestimmungen von klinisch relevanten Parametern gewünschten hohen Genauigkeitsanforderungen sollte bei einer definierten Umschalttemperatur im Bereich zwischen 30 und 40°C die Temperaturabweichung nicht wesentlich über 0,1°C liegen. Die Umschalttemperatur wird durch die Zusammensetzung des leitfähigen Gemisches vorgegeben, während zusätzlich durch geeignete Auslegung der Dicke, Beschaffenheit und Austauschfläche der Testelementfolien die Temperatur am Messort festgelegt wird.

Bei angelegter Spannung ergibt sich unter Stromdurchfluss eine Eigenerwärmung des Heizelements 22. Hieraus resultiert ein Wärmeeintrag in das Untersuchungsfeld 18 und ein Wärmeverlust in die Umgebung, bis eine Phasenübergangstemperatur des leitfähigen Gemisches erreicht wird, die zu einer Volumenänderung und dabei zu einem nichtlinearen sprunghaften Widerstandsanstieg führt.

Für eine hohe Temperaturkonstanz ist eine ausreichende elektrische Versorgungsleistung erforderlich, um den Wärmeverlust auszugleichen. Andererseits wird durch einen gegenüber dem Kaltwiderstand mindestens 100fach erhöhten Heißwiderstand gewährleistet, dass bei Erreichen der Zieltemperatur keine weitere Erwärmung des Gemisches eintritt. Hierdurch erübrigt sich eine gesonderte Vorrichtung zur Temperaturmessung und Steuerung des Heizelements. Grundsätzlich ist es jedoch auch möglich, anhand des momentanen elektrischen Widerstands des Thermistors 22 die Untersuchungstemperatur zu bestimmen, ohne dass ein gesonderter Temperaturfühler erforderlich wäre.

Denkbar ist es auch, eine Ausnehmung in der Zwischenfolie 26 vorzusehen, in welche das leitfähige Gemisch so eingebracht wird, dass diese unmittelbar an das Untersuchungsfeld 18 angrenzt. Weiterhin sind Ausführungsformen möglich, bei denen das Heizfeld 22 seitlich und oben von dem Untersuchungsfeld 18 abgedeckt wird. Ferner sind auch Ausführungen vorstellbar, in denen das Heiz- und Untersuchungsfeld eine integrierte Einheit bilden.

Für die *in-vitro* Diagnostik wird von einem Probanden ein Testelement 12 am Einlassbereich des Kanals 30 mit einem Blutstropfen in Berührung gebracht und sodann in einem Schacht des Handgeräts 10 einer automatischen Verarbeitung unterworfen. Nach Anzeige und gegebenenfalls Speicherung des Messergebnisses wird das Testelement 12 als Verbrauchsmaterial entsorgt.

Als Einsatzmöglichkeit für selbsttemperierbare Testelemente sind auch Einwegkartuschen denkbar, die in kleinen Standgeräten am Patientenbett bzw. in der Arztpraxis beispielsweise für Blutgerinnungstests eingesetzt werden.

## Patentansprüche

1. Testelement zur Untersuchung von Probenmaterial wie Blut oder Urin mit einem Testträger (12), welcher einen mit dem Probenmaterial beaufschlagbaren Untersuchungsbereich (18) aufweist, und einem mit dem Untersuchungsbereich (18) in Wärmeleitkontakt stehenden Heizelement (22), **dadurch gekennzeichnet, dass** das in den Testträger (12) integrierte Heizelement durch einen unter Stromdurchfluss eigenerwärmten, auf eine vorgegebene Zieltemperatur selbstregelnden Thermistor (22) gebildet ist.

2. Testelement nach Anspruch 1, **dadurch gekennzeichnet, dass** der Thermistor (22) als Kaltleiter bei zunehmender Temperatur im Bereich der Zieltemperatur einen sprunghaften, nichtlinearen Widerstandsanstieg aufweist.

3. Testelement nach Anspruch 1 oder 2, **dadurch gekennzeichnet, dass** der Thermistor (22) als Heizfeld, vorzugsweise als Dünnschichtheizfeld ausgebildet ist.

4. Testelement nach einem der Ansprüche 1 bis 3, **dadurch gekennzeichnet, dass** der Thermistor (22) vorzugsweise durch ein Beschichtungs- oder Druckverfahren als Flächengebilde in den Testträger (12) integriert ist.

5. Testelement nach einem der Ansprüche 1 bis 4, **dadurch gekennzeichnet, dass** der Thermistor (22) aus einem Kompositwerkstoff bestehend aus einem Bindemittel und darin eingebrachten elektrisch leitfähigen Bestandteilen gebildet ist.

6. Testelement nach Anspruch 5, **dadurch gekennzeichnet, dass** der Kompositwerkstoff bei der Zieltemperatur einen die elektrische Leitfähigkeit beeinflussenden Phasenübergang durchläuft.

7. Testelement nach Anspruch 5 oder 6, **dadurch gekennzeichnet, dass** das Bindemittel aus Monomeren oder Polymeren besteht

8. Testelement nach einem der Ansprüche 5 bis 7, **dadurch gekennzeichnet, dass** die leitfähigen Bestandteile durch Rußpartikel, Kohlenstofffasern, Metallfäden oder leitfähige Polymerteilchen gebildet sind.

9. Testelement nach einem der Ansprüche 1 bis 8, **dadurch gekennzeichnet, dass** der Testträger (12) als Einwegteil für Einmaluntersuchungen vorgesehen ist.

10. Testelement nach einem der Ansprüche 1 bis 9, **dadurch gekennzeichnet, dass** der Testträger (12) durch ein flächiges Substrat, insbesondere einen vorzugsweise als Folienverbundteil ausgebildeten Teststreifen gebildet ist.

11. Testelement nach einem der Ansprüche 1 bis 10, **dadurch gekennzeichnet, dass** der Untersuchungsbereich (18) zumindest teilweise durch den Thermistor (22) begrenzt ist oder mit diesem über eine Zwischenfolie (26) thermisch leitend verbunden ist.

12. Testelement nach einem der Ansprüche 1 bis 11, **dadurch gekennzeichnet, dass** der Untersuchungsbereich (18) durch ein mit Trockenchemikalien beschichtetes, auf einen Analyten in dem aufgebrachten flüssigen Probenmaterial ansprechendes Reaktionsfeld gebildet ist.

13. Testelement. nach einem der Ansprüche 1 bis 12, **dadurch gekennzeichnet, dass** der Thermistor (22) sich über den Untersuchungsbereich (18) hinaus auf einen Probenzuführkanal (30) des Testträgers (12) zur Vorerwärmung des Probenmaterials erstreckt.

14. Testelement nach einem der Ansprüche 1 bis 13, **dadurch gekennzeichnet, dass** der Thermistor (22) zugleich einen Temperaturfühler zur Bestimmung der Untersuchungstemperatur über eine Widerstandsmessung bildet.

15. Testelement nach einem der Ansprüche 1 bis 14, **dadurch gekennzeichnet, dass** an dem Testträger (12) mit dem Thermistor (22) verbundene, vorzugsweise durch Leiterbahnen gebildete Anschlüsse (40) für eine Spannungsquelle angeordnet sind.

16. Testelement nach einem der Ansprüche 1 bis 15, **dadurch gekennzeichnet, dass** die Zieltemperatur im Bereich zwischen 25 bis 50°C, vorzugsweise 30 bis 40°C liegt.

17. Testelement nach einem der Ansprüche 1 bis 16, **dadurch gekennzeichnet, dass** die Abweichung der Zieltemperatur weniger als 1°C beträgt.

18. Testelement nach einem der Ansprüche 1 bis 17, **dadurch gekennzeichnet, dass** die gesamte Heizzeit weniger als 5 Minuten beträgt.

19. Testelement nach einem der Ansprüche 1 bis 18, **dadurch gekennzeichnet, dass** der Thermistor (22) nur ein Teilvolumen des Probenmaterials, welches sich in Kontakt mit dem Untersuchungsbereich (18) und gegebenenfalls in einem einströmseitig angrenzenden Abschnitt des Probenzuführkanals (30) befindet, direkt erwärmt.

20. Messgerät, insbesondere portables Blutzuckermessgerät(10) oder portables Blutgerinnungsmessgerät zur Verarbeitung von Testelementen nach einem der vorhergehenden Ansprüche.
